(19) Europäisches Patentamt
European Patent Office
Office européen des brevets

(11) Publication number: 0 196 156 B1

# (12) EUROPEAN PATENT SPECIFICATION

(45) Date of publication of patent specification: 07.02.90

(51) Int. Cl.$^5$: C 07 C 69/74, C 07 C 69/65, C 07 C 33/48, A 01 N 53/00, A 01 N 37/10

(21) Application number: 86300921.3

(22) Date of filing: 11.02.86

## (54) Fluorobenzyl esters.

(30) Priority: 06.03.85 GB 8505819

(43) Date of publication of application: 01.10.86 Bulletin 86/40

(45) Publication of the grant of the patent: 07.02.90 Bulletin 90/06

(84) Designated Contracting States: AT BE CH DE FR IT LI LU NL SE

(56) References cited:
EP-A-0 016 513
EP-A-0 031 199
EP-A-0 054 360
EP-A-0 060 617
FR-A-2 096 239

(73) Proprietor: IMPERIAL CHEMICAL INDUSTRIES PLC
Imperial Chemical House Millbank
London SW1P 3JF (GB)

(72) Inventor: Robson, Michael John
12 Greenham Wood North lane
Bracknell Berkshire (GB)
Inventor: Williams, John
36 Wylam Great Hollands
Bracknell Berkshire (GB)

(74) Representative: Bishop, Nigel Douglas et al
Imperial Chemical Industries PLC Legal Department: Patents PO Box 6 Bessemer Road
Welwyn Garden City Herts, AL7 1HD (GB)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 196 156 B1

Courier Press, Leamington Spa, England.

**Description**

1. This invention relates to novel fluorobenzyl esters useful as insecticides and characterised by a combination of excellent knock-down properties and lethal action to insanitary insect pests such as houseflies, cockroaches and mosquitos.

Various 4-substituted fluorobenzyl esters of cyclopropane carboxylic acids are known from EP—A—0 016 513, EP—A—0 031 199 and EP—A—0 060 617. In particular 4-allyl-2,3,5,6-tetrafluorobenzyl esters are known from EP—A—0 031 199. French Patent Publication No. 2 096 239 describes some 4-propargylbenzyl esters lacking fluoro-substitution.

The novel fluorobenzyl esters of this invention have the general formula:

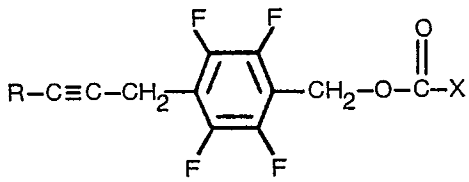

where R is selected from hydrogen, alkyl of up to four carbon atoms and the trialkylsilyl group where each alkyl contains up to four carbon atoms, and X represents

(a) a group of formula:

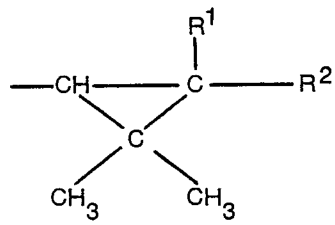

wherein (i) $R^1$ and $R^2$ are each selected from hydrogen, halo and alkyl of up to four carbon atoms; or (ii) $R^1$ is hydrogen and $R^2$ represents either a group of formula:

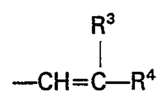

or a group of formula:

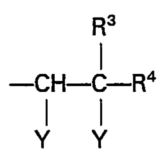

where $R^3$ and $R^4$ are each selected from methyl, halo, and haloalkyl of one or two carbon atoms containing at least two fluorine atoms, and Y is chloro or bromo; or

(b) X represents a group of formula:

$$Ar—CHR^5—$$

where $R^5$ represents an alkyl group of up to four carbon atoms and Ar represents a phenyl group optionally substituted with one or two halogen atoms.

Preferred compounds according to the invention are those of formula:

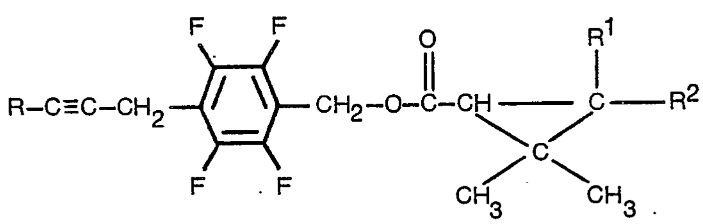

(I)

wherein R is hydrogen or alkyl of up to four carbon atoms, $R^1$ and $R^2$ are each selected from halogen and alkyl of up to four carbons (preferably methyl), or $R^1$ is hydrogen and $R^2$ is a group of formula:

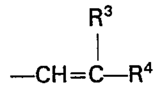

wherein $R^3$ and $R^4$ are each selected from methyl, fluoro, chloro, bromo and trifluoromethyl.

Particularly preferred compounds according to formula I are those set out in Table I below wherein the meaning of $R^1$ and $R^2$ are set out for each compound.

**TABLE I**

| Compound | R | $R^1$ | $R^2$ |
|---|---|---|---|
| 1 | H | $CH_3$ | $CH_3$ |
| 2 | H | H | $-CH=C(CH_3)_2$ |
| 3 | H | H | $-CH=CCl_2$ |
| 4 | H | H | $-CH=CBr_2$ |
| 5 | H | H | $-CH=CF_2$ |
| 6 | H | H | $-CH=C(F)CF_3$ |
| 7 | H | H | $-CH=C(Cl)CF_3$ |
| 8 | H | H | $-CH=C(Br)CF_3$ |
| 9 | H | H | $-CH=C(CF_3)_2$ |
| 10 | H | Cl | Cl |
| 11 | $CH_3$ | H | $-CH=C(Cl)CF_3$ |

It will be appreciated that certain of the compounds of the invention wherein $R^1$ and $R^2$ are not identical are capable of existing in more than one isomeric form, due to the possibility of *cis* and *trans* isomerism in the substitution pattern of the cyclopropane ring and the presence of chiral centres at $C_1$ and $C_3$ of the cyclopropane ring. Thus there may be (+)-*cis*, (−)-*cis*, (+)-*trans* and (−)-*trans* isomers. Where $R^3$ and $R^4$ are not identical there exists the further possibility of E and Z isomers of the group $R^2$. The scope of the invention includes each of the said isomeric forms in isolation as well as mixtures thereof, including racemic mixtures.

Especially preferred compounds according to formula I include the 4-prop-2-ynyl-2,3,5,6-tetrafluorobenzyl esters of the following cyclopropane acids.

2,2,3,3-tetramethylcyclopropane carboxylic acid,
2,2-dichloro-3,3-dimethylcyclopropane carboxylic acid,
(±)-*cis*-3-(2,2-dichlorovinyl)-2,2-dimethylcyclopropane carboxylic acid,
(±)-*trans*-3-(2-methylprop-1-en-1-yl)-2,2-dimethylcyclopropane carboxylic acid,
(±)-*cis*-3-(2,2-difluorovinyl)-2,2-dimethylcyclopropane carboxylic acid,
(±)-*cis*-3-(2,2-dibromovinyl)-2,2-dimethylcyclopropane carboxylic acid,
(+)-*cis*-3-(2,2-dibromovinyl)-2,2-dimethylcyclopropane carboxylic acid,
(±)-*cis*-3-(Z-2-chloro-3,3,3-trifluoroprop-1-en-1-yl)-2,2-dimethylcyclopropane carboxylic acid,
(+)-*cis*-3-(Z-2-chloro-3,3,3-trifluoroprop-1-en-1-yl)-2,2-dimethylcyclopropane carboxylic acid,
(±)-*cis*-3-(Z-2,3,3,3-tetrafluoroprop-1-en-1-yl)-2,2-dimethylcyclopropane carboxylic acid,
(±)-*trans*-3-(Z-2,3,3,3-tetrafluoroprop-1-en-1-yl)-2,2-dimethylcyclopropane carboxylic acid, and
(±)-*cis*-3-(2-trifluoromethyl-3,3,3-trifluoroprop-1-en-1-yl)-2,2-dimethylcyclopropane carboxylic acid.

Other compounds of the invention include

4-but-2-ynyl-2,3,5,6-tetrafluorobenzyl (±)-*cis*-3-(Z-2-chloro-3,3,3-trifluoroprop-1-en-1-yl)-2,2-dimethylcyclopropane carboxylate, and the 4-prop-2-ynyl-2,3,5,6-tetrafluorobenzyl esters of (±)-*cis*-3-(1,2-dibromo-2,2-dichloromethyl)-2,2-dimethylcyclopropane carboxylic acid, and (±)-2-(4-chloro-phenyl)-3-methylbutyric acid.

The compounds of the invention are esters and may be prepared by conventional esterification processes, of which the following are examples:

(a) An acid of formula

$$X—COOH \qquad (II)$$

where X has any of the meanings given hereinabove, may be reacted directly with a 4-alkynyl-2,3,5,6-tetrafluorobenzyl alcohol of formula:

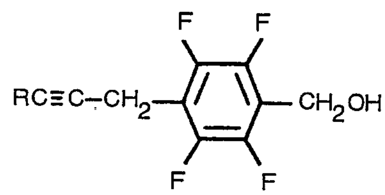

(III)

the reaction preferably taking place in the presence of an acid catalyst, for example, dry hydrogen chloride, or a dehydrating agent, for example, a carbodiimide such as dicyclohexylcarbodiimide.

(b) An acid halide of formula X—COHal where Hal represents a halogen atom, preferably a chlorine atom, and X has any of the meanings given hereinabove, may be reacted with the alcohol of formula III, the reaction preferably taking place in the presence or a base, for example, pyridine, trialkylamine alkali metal hydroxide or carbonate, or alkali metal alkoxide.

(c) An acid of formula II where X has any of the meanings given hereinabove, or preferably, an alkali metal salt thereof, may be reacted with a halide of formula:

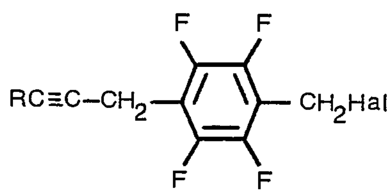

(IV)

where Hal represents a halogen atom, preferably the bromine or chlorine atom, or with the quaternary ammonium salts derived from such halides with tertiary amines, for example pyridine, or trialkylamines such as triethylamine.

(d) A lower alkyl ester of formula X—COOQ where Q represents a lower alkyl group containing up to six carbon atoms, preferably the methyl or ethyl group, and X has any of the meanings given hereinabove, is heated with the alcohol of formula III to effect a transesterification reaction. Preferably the process is performed in the presence of a suitable catalyst, for example, an alkali metal alkoxide, such as sodium methoxide, or an alkylated titanium derivative, such as tetramethyl titanate.

All of these conventional processes for the preparation of esters may be carried out using solvents and diluents for the various reactants where appropriate, and may be accelerated or lead to higher yields of product when performed at elevated temperatures or in the presence of appropriate catalysts, for example phase-transfer catalysts.

The preparation of individual isomers may be carried out in the same manner but commencing from the corresponding individual isomers of compounds of formula II. These may be obtained by conventional isomer separation techniques from mixtures of isomers. Thus *cis* and *trans* isomers may be separated by fractional crystallisation of the carboxylic acids or salts thereof, whilst the various optically active species may be obtained by fractional crystallisation of salts of the acids with optically active amines, followed by regeneration of the optically pure acid. The optically pure isomeric form of the acid (or its equivalent acid chloride or ester) may then be reacted with the alcohol of formula III to produce a compound of formula I in the form of an individually pure isomer thereof.

4-Alkynyl-2,3,5,6-tetrafluorobenzyl alcohols (III) have not been described previously. In a further aspect therefore the invention provides 4-alkynyl-2,3,5,6-tetrafluorobenzyl alcohols as novel intermediates useful in the preparation of the insecticidal esters of the invention.

These compounds may be prepared from pentafluorobenzaldehyde by the following scheme.

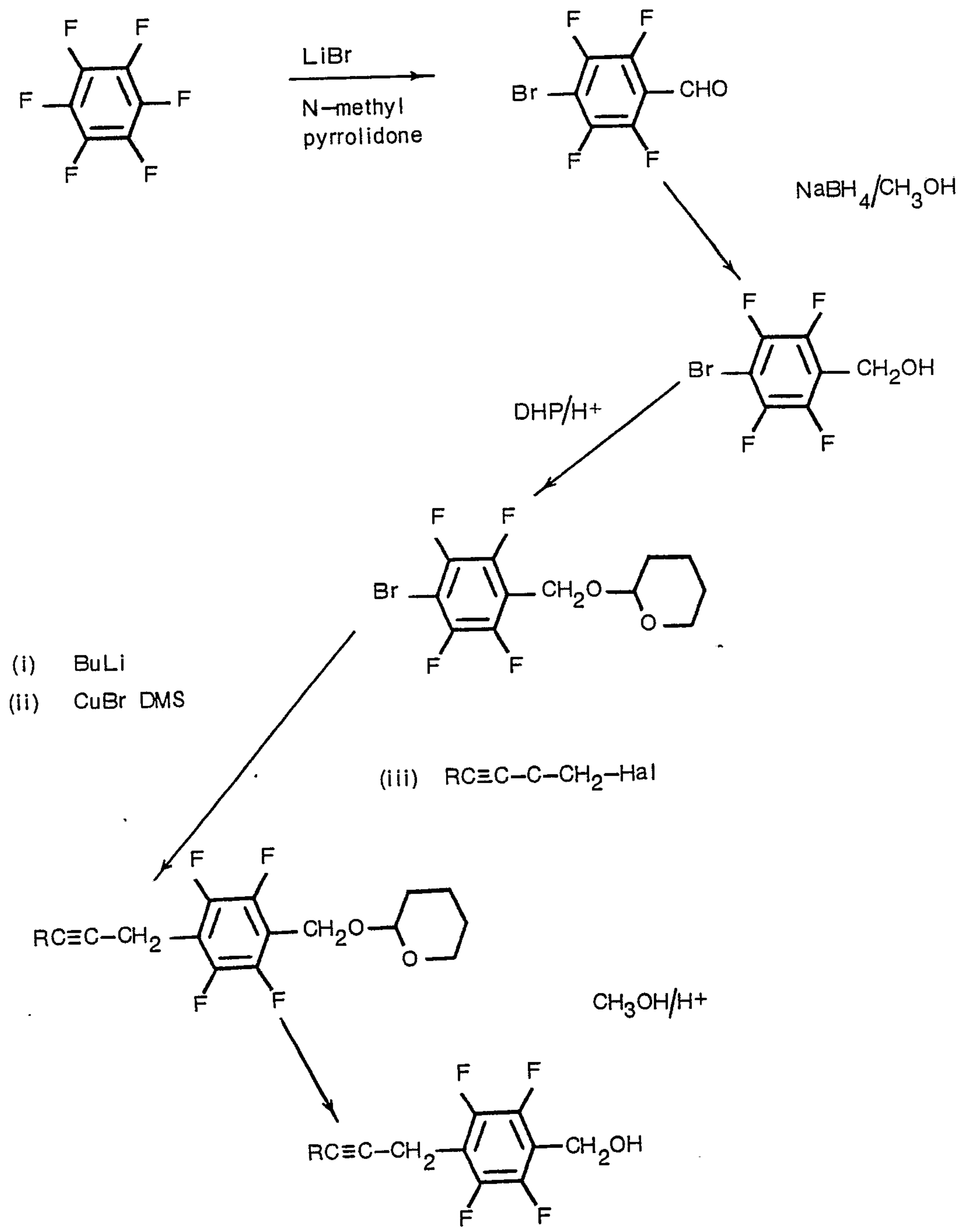


Key: DHP = dihydropyran; BuLi = *n*-butyllithium
CuBr-DMS = copper (I) bromide, complex with dimethylsulphide

Further details concerning the preparation and characterisation of 4-prop-2-ynyl-2,3,5,6-tetrafluorobenzyl alcohol and 4-but-2-ynyl-2,3,5,6-tetrafluorobenzyl alcohols are given hereinafter in the Examples.

The compounds of formula I may be used to combat and control infestation of insect pests and also other invertebrate pests, in particular, acarine pests. The insect and acarine pests which may be combatted and controlled by the use of the invention compounds include those pests associated with agriculture (which term includes the growing of crops for food and fibre products, horticulture and animal husbandry), forestry, the storage of products of vegetable origin, such as fruit, grain and timber, and also those pests associated with the transmission of diseases of man and animals.

In order to apply the compounds to the locus of the pests they are usually formulated into compositions which include in addition to the insecticidally active ingredient or ingredients of formula I suitable inert diluent or carrier materials, and/or surface active agents. The compositions may also comprise another pesticidal material, for example another insecticide or acaricide, or a fungicide, or may

also comprise a insecticide synergist, such as for example dodecyl imidazole, safroxan, or piperonyl butoxide.

The compositions may be in the form of dusting powders wherein the active ingredient is mixed with a solid diluent or carrier, for example kaolin, bentonite, kieselguhr, or talc, or they may be in the form of granules, wherein the active ingredient is absorbed in a porous granular material for example pumice.

Alternatively the compositions may be in the form of liquid preparations to be used as dips or sprays, which are generally aqueous dispersions or emulsions of the active ingredient in the presence of one or more known wetting agents, dispersing agents or emulsifying agents (surface active agents).

Wetting agents, dispersing agents and emulsifying agents may be of the cationic, anionic or non-ionic type. Suitable agents of the cationic type include, for example, quaternary ammonium compounds, for example cetyltrimethyl ammonium bromide. Suitable agents of the anionic type include, for example, soaps, salts of aliphatic monoesters of sulphuric acid, for example sodium lauryl sulphate, salts of sulphonated aromatic compounds, for example sodium dodecylbenzenesulphonate, sodium, calcium or ammonium lignosulphonate, or butylnaphthalene sulphonate, and a mixture of the sodium salts of diisopropyl- and triisopropylnaphthalene sulphonates. Suitable agents of the non-ionic type include, for example, the condensation products of ethylene oxide with fatty alcohols such as oleyl alcohol or cetyl alcohol, or with alkyl phenols such as octyl phenol, nonyl phenol and octyl cresol. Other non-ionic agents are the partial esters derived from long chain fatty acids and hexitol anhydrides, the condensation products of the said partial esters with ethylene oxide, and the lecithins.

The compositions may be prepared by dissolving the active ingredient in a suitable solvent, for example, a ketonic solvent such as diacetone alcohol, or an aromatic solvent such as trimethylbenzene and adding the mixture so obtained to water which may contain one or more known wetting, dispersing or emulsifying agents. Other suitable organic solvents are dimethyl formamide, ethylene dichloride, isopropyl alcohol, propylene glycol and other glycols, diacetone alcohol, toluene, kerosene, white oil, methylnaphthalene, xylenes and trichloroethylene, N-methyl-2-pyrrolidone and tetrahydro furfuryl alcohol (THFA).

The compositions to be used as sprays may also be in the form of aerosols wherein the formulation is held in a container under pressure in the presence of a propellant such as fluorotrichloromethane or dichlorodifluoromethane.

The compositions which are to be used in the form of aqueous dispersions or emulsions are generally supplied in the form of a concentrate containing a high proportion of the active ingredient or ingredients, the said concentrate to be diluted with water before use. These concentrates are often required to withstand storage for prolonged periods and after such storage, to be capable of dilution with water to form aqueous preparations which remain homogenous for a sufficient time to enable them to be applied by conventional spray equipment. The concentrates may contain 10—85% by weight of the active ingredient or ingredients. When diluted to form aqueous preparations such preparations may contain varying amounts of the active ingredient depending upon the purpose for which they are to be used. For agricultural or horticultural purposes, an aqueous preparation containing between 0.0001% and 0.1% by weight of the active ingredient is particularly useful.

In use the compositions are applied to the pests, to the locus of the pests, to the habitat of the pests, or to growing plants liable to infestation by the pests, by any of the known means of applying pesticidal compositions, for example, by dusting or spraying.

The compositions of the invention are very toxic to wide varieties of insect and other invertebrate pests, including, for example, the following:

*Aphis fabae* (aphids)
*Megoura viceae* (aphids)
*Musca domestica* (houseflies)
*Blattella germanica* (cockroaches)
*Aedes aegypti* (mosquitos)
*Plutella xylostella* (diamond back moth, larvae)
*Tetranychus cinnabarinus* (carmine spider mite)
*Tetranychus urticae* (red spider mites)
*Panonychus ulmi* (citrus rust mite)
*Diabrotica* spp. (rootworms)
*Heliothis virescens* (tobacco budworms)
*Chilo partellus* (stem borers)

The compounds of formula I and compositions comprising them have shown themselves to be particularly useful in controlling foliar feeding pests of plants, and public health pests such as flies and mosquitos. The compounds may also be used to combat pests which inhabit the soil, for example *Diabrotica* spp. They may also be useful in combating insect and acarine pests which infest domestic animals, such as *Lucilia sericata,* and ixodid ticks such as *Boophilus* spp., *Ixodes* spp., *Amblyomma* spp., *Rhipicephalus* spp., and *Dermaceutor* spp. They are expected to be effective in combating both susceptible and resistant strains of these pests in their adult, larval and intermediate stages of growth, and may be applied to the infested host animal by topical, oral or parenteral administration.

In particular, the invention compounds have useful knock-down properties which make them of value

as ingredient in aerosol and like sprays designed for use against flying insect pests such as houseflies, mosquitos and the like. In such sprays they may be used on their own or in conjunction with other insecticidal components. However, unlike other knock-down agents such as tetramethrin and allethrin many of the invention compounds combine good knock-down properties with a lethal effect at the same concentration.

In the following examples, which further illustrate the invention, the novel compounds produced were examined by infra-red and nuclear-magnetic spectroscopy, and gave spectra consistent with the indicated structures of the compounds.

Example 1

This Example illustrates the preparation of 4-bromo-2,3,5,6-tetrafluorobenzaldehyde.

A stirred mixture of pentafluorobenzaldehyde (17.7 g), anhydrous lithium bromide (8.9 g) and N-methylpyrrolidone (50 $cm^3$) was heated at 160°C under a nitrogen atmosphere for 2 hours, after which it was cooled and poured into water. The solid precipitate was collected by filtration, washed on the filter with water and dried in a dessicator over phosphorus pentoxide. After trituration with diethyl ether the residual solid was collected to yield 4-bromo-pentafluorobenzaldehyde (8.4 g), mp. 105—108°C.

Infra red (paraffin mull): 1700 $cm^{-1}$

Example 2

This Example illustrates the preparation of 4-bromo-2,3,5,6-tetrafluorobenzyl alcohol.

Sodium borohydride (1.0 g) was added portionwise over a period of 30 minutes to a stirred solution of 4-bromo-2,3,5,6-tetrafluorobenzaldehyde (8.2 g) in methanol (80 $cm^3$) whilst the temperature was maintained within the range from −5°C to +5°C, after which the mixture was stirred for 2 hours at the ambient temperature (ca 18°C). The mixture was poured into water and the precipitated white solid collected by filtration, washed with water and air dried to yield 4-bromo-2,3,5,6-tetrafluorobenzyl alcohol (7.5 g), mp. 60—62°C.

Infra red (paraffin mull): 3400 (b), 1500 (b) $cm^{-1}$

Example 3

This Example illustrates the preparation of 2-(4-bromo-2,3,5,6-tetrafluorobenzyloxy)tetrahydrofuran.

Dihydropyran (3.0 g) and concentrated hydrochloric acid (0.3 $cm^3$) were added successively to a stirred solution of 4-bromo-2,3,5,6-tetrahydrobenzyl alcohol (8.4 g) in dry diethyl ether (50 $cm^3$) and the mixture stirred for a further 15 hours after which the more volatile components were removed by evaporation under reduced pressure. The residual oil was confirmed by spectroscopic analysis as being 2-(4-bromo-2,3,5,6-tetrafluorobenzyloxy)tetrahydropyran (9.5 g) of ca 95% purity.

$^1H$ NMR ($CDCl_3$) δ: 4.6 (m, 3H); 3.9 (m, 2H); 1.6 (m, 6H).

Example 4

This Example illustrates the preparation of 2-(4-prop-2-ynyl-2,3,5,6-tetrafluorobenzyloxy)tetrahydropyran.

*n*-Butyllithium (2.3 $cm^3$ of a 2.5M solution in *n*-hexane) was added dropwise to a solution of 2-(4-bromo-2,3,5,6-tetrafluorobenzyloxy)tetrahydropyran (1.7 g) in dry tetrahydrofuran (50 $cm^3$) maintained at a temperature of −70°C under a nitrogen atmosphere, and the resulting mixture stirred for a further 2 hours. Copper (I) bromidedimethylsulphide complex (1.1 g) was added to the mixture and after stirring for a further 2 hours, propargyl chloride (0.4 g) was added dropwise to the clear brownish-yellow solution. The mixture was maintained at −70°C for 1 hour and then allowed to warm to the ambient temperature over a period of 4 hours. The mixture was partitioned between diethyl ether and aqueous saturated ammonium chloride solution, and the ethereal phase separated, washed with water and dried over anhydrous magnesium sulphate. Removal of the solvent by evaporation under reduced pressure yielded a residual oil (1.4 g) consisting of ca. 60% of the desired product together with ca 20% 2-(2,3,5,6-tetrafluorobenzyloxy)tetrahydropyran, and ca. 20% other unidentified materials. Purification and separation was effected by means of hplc (Gilson) using a silica column eluted with a mixture of *n*-hexane (9 parts by volume) and diethyl ether (1 part by volume) to yield pure 2-(4-prop-2-ynyl-2,3,5,6-tetrafluorobenzyloxy)-tetrahydropyran (0.8 g).

$^1H$ NMR ($CDCl_3$) δ: 4.6 (m, 3H); 3.9 (m, 2H); 3.6 (d, 2H); 2.0 (t, 1H); 1.6 (m, 6H)

Infra red (liquid film): 3300, 2700 $cm^{-1}$

Example 5

This Example illustrates the preparation of 4-prop-2-ynyl-2,3,5,6-tetrafluorobenzyl alcohol.

A mixture of 2-(4-prop-2-ynyl-2,3,5,6-tetrafluorobenzyloxy)tetrahydropyran (0.8 g), dilute hydrochloric acid (2N, 5 $cm^3$) and methanol (30 $cm^3$) was stirred together at the ambient temperature for 2 hours, after which the more volatile portion was removed by evaporation under reduced pressure. The residue was extracted with diethyl ether, the extracts combined, washed with water, and dried over anhydrous magnesium sulphate. Removal of the solvent by evaporation under reduced pressure yielded an oil which crystallised on standing to give 4-prop-2-ynyl-2,3,5,6-tetrafluorobenzyl alcohol (0.5 g), mp. 51—52°C, after recrystallisation from petroleum ether (boiling range 60—80°C).

$^1H$ NMR ($CDCl_3$) δ: 4.8 (s, 2H); 3.6 (m, 2H); 2.3 (broad s, 1H); 1.0 (t, 1H)
Infra red (paraffin mull): 3400 $cm^{-1}$.

Example 6

This Example illustrates the preparation of 4-prop-2-ynyl-2,3,5,6-tetrafluorobenzyl (±)-*cis*-3-(Z-2-chloro-3,3,3-trifluoroprop-1-en-1-yl)-2,2-dimethylcyclopropane carboxylate (Product I).

A mixture of (±)-*cis*-3-(Z-2-chloro-3,3,3-trifluoroprop-1-en-1-yl)-2,2-dimethylcyclopropane carboxylic acid (0.12 g), 4-prop-2-ynyl-2,3,5,6-tetrafluorobenzyl alcohol (0.12 g), dicyclohexylcarbodiimide (0.134 g), dichloromethane (10 $cm^3$) and a trace of N,N-dimethylaminopyridine was stirred at the ambient temperature for 1 hour. After removal of the solid component by filtration the filtrate was concentrated by evaporation of the solvent and the residual oil subjected to purification by flash chromatography using dichloromethane as eluant to yield pure 4-prop-2-ynyl-2,3,5,6-tetrafluorobenzyl (±)-*cis*-3-(Z-2-chloro-3,3,3-trifluoroprop-1-en-1-yl)-2,2-dimethylcyclopropane carboxylate (150 mg).

$^1H$ NMR ($CDCl_3$) δ: 6.9 (d, 1H); 5.2 (s, 2H); 3.6 (m, 2H); 2.0 (m, 3H); 1.35 (s, 6H)
Infra red (liquid film): 3300, 1730, 1650 $cm^{-1}$.

Example 7

By the use of a procedure similar to that illustrated in Example 6 the following compounds of formula I may also be prepared from the appropriate carboxylic acids.

(i) 4-prop-2-ynyl-2,3,5,6-tetrafluorobenzyl (±) *cis/trans*-3-(2-methylprop-1-en-1-yl)-2,2-dimethylcyclopropane carboxylate (*cis:trans* ratio 3:7) — Product II.
$^1H$ NMR ($CDCl_3$) δ: [5.3 (d), 5.2 (s), 4.2 (d)-3H)]; 3.6 (m, 2H); 2.0 (t, 1H); 1.7 (s, 6H); 1.2 (dd, 6H).
Infra red (liquid film): 3300, 1730 $cm^{-1}$.

(ii) 4-prop-2-ynyl-2,3,5,6-tetrafluorobenzyl (±)-*cis*-3-(2,2-dichlorovinyl)-2,2-dimethylcyclopropane carboxylate — Product III
$^1H$ NMR ($CDCl_3$) δ: 6.2 (d, 2H); 5.2 (d, 2H); 3.6 (m, 2H); 2.0 (m, 3H); 1.2 (s, 6H)
Infra red (liquid film): 3300, 1730, 1650 $cm^{-1}$.

(iii) 4-prop-2-ynyl-2,3,5,6-tetrafluorobenzyl (±)-*trans*-3-(Z-2-chloro-3,3,3-trifluoroprop-1-en-1-yl)-2,2-dimethylcyclopropane carboxylate — Product IV.
$^1H$ NMR ($CDCl_3$) δ: 6.1 (d, 1H); 5.25 (s, 2H); 3.6 (q, 2H); 2.4 (m, 1H); 2.0 (t, 1H); 1.7 (d, 1H); 1.3 (d, 6H)
Infra red (liquid film): 3300, 1730, 1650 $cm^{-1}$.

(iv) 4-prop-2-ynyl-2,3,5,6-tetrafluorobenzyl 2,2,3,3-tetramethylcyclopropane carboxylate — Product V.
$^1H$ NMR ($CDCl_3$) δ: 5.1 (s, 2H); 3.6 (m, 2H); 2.0 (t, 1H); 1.2 (d, 13H).

(v) 4-prop-2-ynyl-2,3,5,6-tetrafluorobenzyl (+)-*cis*-3-(Z-2-chloro-3,3,3-trifluoroprop-1-en-1-yl)-2,2-dimethylcyclopropane carboxylate — Product VI.
$^1H$ NMR ($CDCl_3$) δ: 6.9 (d, 1H); 5.25 (s, 2H); 3.2 (m, 2H); 2.0 (m, 3H); 1.3 (s, 6H).

(vi) 4-prop-2-ynyl-2,3,5,6-tetrafluorobenzyl (±)-*cis*-3-(2-bromo-3,3,3-trifluoroprop-1-en-1-yl)-2,2-dimethylcyclopropane carboxylate — Product VII.
$^1H$ NMR ($CDCl_3$) δ: 6.9 (d, 1H); 5.08 (s, 2H); 3.5 (m, 2H); 1.93 (m, 3H); 1.16 (s, 6H).
Infra red (liquid film): 3300, 1730 $cm^{-1}$

(vii) 4-prop-2-ynyl-2,3,5,6-tetrafluorobenzyl (±)-3-(1,2-dibromo-2,2-dichloroethyl)-2,2-dimethylcyclopropane carboxylate — Product VIII.
$^1H$ NMR ($CDCl_3$) δ: 5.4 (m, 1H); 5.2 (s, 2H); 3.6 (m, 2H); 2.0 (m, 3H); 1.3 (d, 6H).

(viii) 4-prop-2-ynyl-2,3,5,6-tetrafluorobenzyl (±)-2-(4-chlorophenyl)-3-methylbutyrate — Product IX.
$^1H$ NMR ($CDCl_3$) δ: 5.17 (m, 2H); 3.6 (m, 2H); 3.1 (d, 1H); 2.3 (m, 1H); 2.07 (t, 1H); 1.04 (d, 3H); 0.75 (d, 3H).
Infra red (liquid film): 3300, 1730 $cm^{-1}$.

Example 8

This Example illustrates the preparation of the 4-prop-2-ynyl-2,3,5,6-tetrafluorobenzyl esters of (±)-*cis* and (±)-*trans*-3-(Z-2,3,3,3-tetrafluoroprop-1-en-1-yl)-2,2-dimethylcyclopropane carboxylic acids.

Dicyclohexylcarbodiimide (0.4 g) was added to a stirred mixture of 3-(Z-2,3,3,3-tetrafluoroprop-1-en-1-yl)-2,2-dimethylcyclopropane carboxylic acid (0.45 g, 88% w/w (±)-*cis* isomer, 12% w/w (±)-*trans* isomer), 4-prop-2-ynyl-2,3,5,6-tetrafluorobenzyl alcohol (0.42 g), 4-dimethylaminopyridine (0.01 g) and dichloromethane (10.0 $cm^3$) and the mixture stirred for 1 hour. After removal of the solid component by filtration the filtrate was concentrated by evaporation of the solvent. The residual oil was subjected to purification by h.p.l.c. (Gilson), using a silica gel column eluted with a 1:1 by volume mixture of petroleum ether (boiling range 40—60°C and dichloromethane to yield 4-prop-2-ynyl-2,3,5,6-tetrafluorobenzyl (±)-*cis*-(Z-2,3,3,3-tetrafluoroprop-1-en-1-yl)-2,2-dimethylcyclopropane carboxylate (A, Product X, 400 mg) and 4-prop-2-ynyl-2,3,5,6-tetrafluorobenzyl (±)-*trans*-3-(Z-2,3,3,3-tetrafluoroprop-1-en-1-yl)-2,2-dimethylcyclopropane carboxylate (B, Product XI, 91 mg).

$^1H$ NMR ($CDCl_3$) δ: (A)— 6.2 (d, 0.5H); 5.9 (d, 0.5H); 5.2 (s, 2H); 3.63 (m, 2H); 2.07 (m, 3H); 1.27 (s, 6H)
(B)— 5.7 (d, 0.5H); 5.1 (d, 0.5H); 5.3 (s, 2H); 3.6 (m, 2H); 2.4 (m, 1H); 2.05 (t, 1H); 1.6 (d, 1H); 1.25 (d, 6H).

Example 9

By the use of procedures similar to those described in Examples 4 and 5 above 2-(4-but-2-ynyl-2,3,5,6-tetrafluorobenzyloxy)tetrahydropyran (C) was obtained from 2-(4-bromo-2,3,5,6-tetrafluorobenzyloxy)tetrahydropyran and but-2-ynyl bromide and converted to 4-but-2-ynyl)-2,3,5,6-tetrafluorobenzyl alcohol (D).

$^1$H NMR ($CDCl_3$) δ: (D)— 4.8 (d, 2H); 3.6 (s, 2H); 2.5 (bs, 1H); 1.8 (t, 3H).

Infra red (liquid film): 3400 $cm^{-1}$

Example 10

By the use of a procedure similar to that illustrated in Example 6 the alcohol (D) was converted to 4-but-2-ynyl-2,3,5,6-tetrafluorobenzyl (±)-*cis*-3-(Z-2-chloro-3,3,3-trifluoroprop-1-en-1-yl)-2,2-dimethylcyclopropane carboxylate (Product XII).

$^1$H NMR ($CDCl_3$) δ: 6.9 (d, 1H); 5.25 (s, 2H); 3.6 (bs, 2H); 2.1 (m, 2H); 1.8 (t, 3H); 1.3 (s, 6H).

Infra red (liquid film): 1730, 1650 $cm^{-1}$

Example 11

This Example illustrates the preparation of 3-bromo-1-trimethylsilylpropane.

A solution of triphenylphosphine (2.7 g) in dry diethyl ether (30 $cm^3$) was added dropwise to a stirred mixture of 1-trimethylsilylpropyn-3-ol (1.19 g) and 1,2-dibromotetrachloroethane (3.38 g) and sodium dried diethyl ether (30 $cm^3$) over a period of 8 minutes whilst the temperature was maintained at within the range −3 to +3°C. After stirring for a further 10 minutes the solid was removed by filtration and the filtrate concentrated by evaporation of the solvent under reduced pressure. The residual oil was purified by distillation to yield 3-bromo-1-trimethylsilylpropyne (0.9 g) as a colourless oil (bp. 68°C/20 mm Hg).

$^1$H NMR ($CDCl_3$) δ: 0.2 (s, 9H); 3.9 (s, 2H).

Infra red (liquid film): 2200 $cm^{-1}$

Example 12

By the use of procedures similar to those described in Examples 4 and 5 above 2-(4-trimethylsilylprop-2-ynyl-2,3,5,6-tetrafluorobenzyloxy)tetrahydropyran (E) was obtained from 3-bromo-1-trimethylsilylpropyne and 2-(4-bromo-2,3,5,6-tetrafluorobenzyloxy)tetrahydropyran, and converted to 4-trimethylsilylprop-2-ynyl-2,3,5,6-tetrafluorobenzyl alcohol (F).

$^1$H NMR ($CDCl_3$) δ: (F)— 4.8 (d, 2H); 3.6 (s, 2H); 0.1 (s, 9H).

Infra red (liquid film): 3400, 2200 $cm^{-1}$

Example 13

By the use of a procedure similar to that illustrated in Example 6 the alcohol (F) was converted to 4-trimethylsilylprop-2-ynyl-2,3,5,6-tetrafluorobenzyl (±)-*cis*-3-(2,2-dichlorovinyl)-2,2-dimethylcyclopropane carboxylate (Product XIII).

$^1$H NMR ($CDCl_3$) δ: 6.3 (d, 1H); 5.25 (d, 2H); 3.6 (bs, 2H); 2.0 (m, 2H); 1.3 (s, 6H); 0.1 (s, 9H).

Infra red (liquid film): 2200, 1730, 1650 $cm^{-1}$

Example 14

This Example illustrates the insecticidal properties of the Products of this invention.

The activity of the Product was determined using a variety of insect pests. The Product was used in the form of liquid preparations containing 500, 100 or 10 parts per million (ppm) by weight of the Product. The preparations were made by dissolving the Product in a mixture of solvents consisting of 4 parts by volume of acetone and 1 part by volume of diacetone alcohol. The solutions were then diluted with water containing 0.01% by weight of a wetting agent sold under the trade name "LISSAPOL" NX until the liquid preparations contained the required concentration of the Product. "Lissapol" is a Registered Trade Mark.

The test procedure adopted with regard to each pest was basically the same and comprised supporting a number of the pests on a medium which was usually a host plant or a foodstuff on which the pests feed, and treating either or both the pests and the medium with the preparations. The mortality of the pests was then assessed at periods usually varying from one to three days after the treatment. Details are given in Table II.

The results of the tests are given in Table III for each of the Products at the rate in parts per million given in the second column as a grading of mortality designated as A, B, C or N wherein A indicates 100% mortality of at an application rate of 10 ppm, B indicates 100% mortality at 100 ppm, C indicates 100% mortality at 500 ppm and N represents less than 100% mortality at 500 ppm.

In Table III the pest organism used is designated by a letter code and the pest species, the support medium or food, and the type and duration of test is given in Table II.

TABLE II

| Code Letters (Table III) | Test Species | Support Medium/Food | Type of Test | Duration (days) |
|---|---|---|---|---|
| TU | *Tetranychus urticae* (red spider mites — adult) | French bean leaf | Contact | 3 |
| MP | *Myzus persicae* (aphids) | leaf | Contact | 2 |
| NL | *Nilaparvata lugens* (Green leaf hopper) | Cabbage leaf | Contact | 6 |
| CP | *Chilo partellus* (maize stem borer) | Oil seed rape leaf | Residual | 3 |
| HV | *Heliothis viriscens* (tobacco budworm) | Cotton leaf | Residual | 3 |
| DB | *Diabrotica balteata* (rootworm larvae) | Filter paper/maize seed | Residual | 3 |
| BG | *Blattella germanica* (cockroach nymphs) | Plastic pot | Residual | 3 |
| MD | *Musca domestica* (houseflies — adults) | Cotton wool/sugar | Contact | 1 |

"Contact" test indicates that both pests and medium were treated and "residual" indicates that the medium was treated before infestation with the pests.

TABLE III

| PRODUCT | TU | MP | NL | MD | BG | HV | CP | DB |
|---|---|---|---|---|---|---|---|---|
| I | A | A | B | A | A | B | A | A |
| II | B | A | C | B | B | B | A | A |
| III | A | B | N | A | A | A | A | B |
| IV | A | A | N | A | A | A | A | A |
| V | C | A | B | B | B | B | A | A |
| X | - | B | C | B | B | B | A | A |
| XI | B | B | C | A | B | B | A | A |
| XII | B | A | B | B | B | A | A | A |
| XIII | B | C | N | C | C | C | C | A |

In the test against *Musca domestica* the knock-down effect was observed. Products I, III, IV and XI gave the most rapid knock-down.

**Claims**

1. An ester of formula:

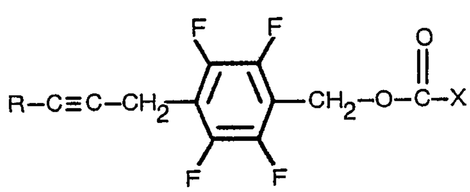

where R is selected from hydrogen, alkyl of up to 4 carbon atoms and trialkylsilyl where each alkyl group contains up to 4 carbon atoms, and X represents

(a) a group of formula:

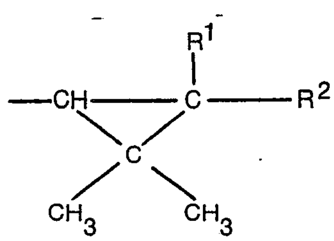

wherein (i) $R^1$ and $R^2$ are each selected from hydrogen, halo and alkyl of up to four carbon atoms; or (ii) $R^1$ is hydrogen and $R^2$ represents either a group of formula:

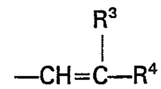

or a group of formula:

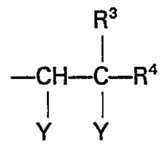

where $R^3$ and $R^4$ are each selected from methyl, halo, and haloalkyl of one or two carbon atoms containing at least two fluorine atoms, and Y is chlorine or bromo; or

(b) X represents a group of formula:

$$Ar—CHR^5—$$

where $R^5$ represents an alkyl group of up to four carbon atoms and Ar represent a phenyl group optionally substituted with one or two halogen atoms.

2. An ester according to claim 1 of formula:

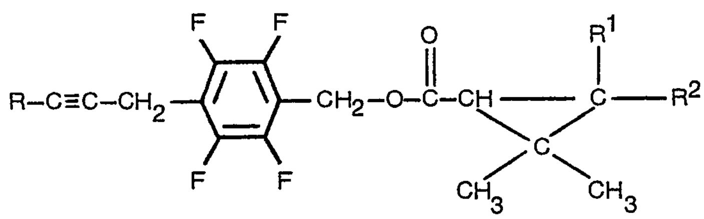

wherein R is hydrogen or alkyl of up to four carbon atoms, and $R^1$ and $R^2$ are each selected from halogen and alkyl of up to four carbons atoms, or $R^1$ is hydrogen and $R^2$ is a group of formula:

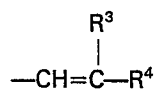

wherein $R^3$ and $R^4$ are selected from methyl, fluoro, chloro, bromo and trifluoromethyl.

3. The ester according to claim 2 wherein R is hydrogen, $R^1$ is hydrogen and $R^2$ is dichlorovinyl or 2-chloro-3,3,3-trifluoroprop-1-en-1-yl.

4. An insecticidal composition comprising an insecticidally effective amount of the ester of claim 1 in association with an insecticidally inert diluent or carrier material.

5. A method of combating insect pests at a locus which comprises treating the locus with an insecticidally effective amount of the composition of claim 4.

6. The method of claim 5 wherein the insect pests are flying insect pests and the composition comprises an amount of the compound of claim 1 sufficient to cause rapid knock-down of the pests.

7. A process for preparing the ester of claim 1 which comprises reacting an acid of formula X—COOH, or an esterifyable derivative thereof, with an alcohol of formula:

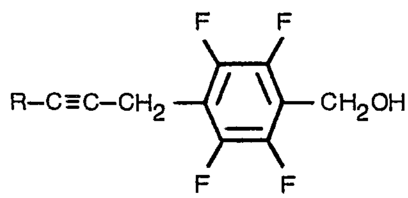

wherein X and R are as defined in claim 1.

8. An alcohol of formula:

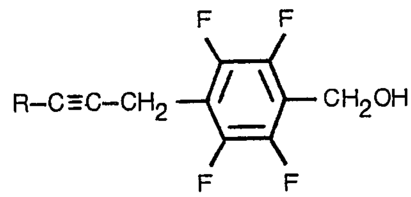

wherein R is hydrogen or alkyl of up to four carbon atoms.

**Patentansprüche**

1. Ester der Formel

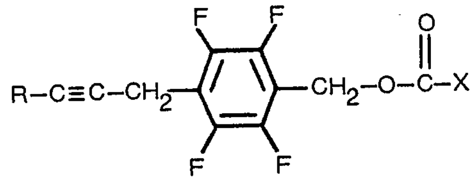

worin R ausgewählt ist aus Wasserstoff, Alkyl mit bis zu 4 Kohlenstoffatomen und Trialkylsilyl, wobei jede Alkylgruppe bis zu 4 Kohlenstoffatomen enthält, und X für folgendes steht:

(a) eine Gruppe der Formel

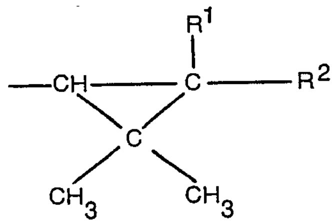

worin (i) $R^1$ und $R^2$ jeweils ausgewählt sind aus Wasserstoff, Halogen und Alkyl mit bis zu 4 Kohlenstoffatomen oder (ii) $R^1$ für Wasserstoff steht und $R^2$ entweder für eine Gruppe der Formel

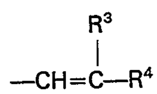

oder eine Gruppe der Formel

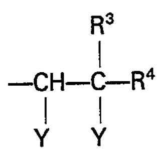

steht, worin $R^3$ und $R^4$ jeweils ausgewählt sind aus Methyl, Halogen und Halogenalkyl mit einem oder zwei Kohlenstoffatomen und mit mindestens zwei Fluoratomen, und Y für Chlor oder Brom steht; oder

(b) eine Gruppe der Formel

Ar—CHR$^5$—

worin $R^5$ für eine Alkylgruppe mit bis zu 4 Kohlenstoffatomen steht und Ar für eine Phenylgruppe, die gegebenenfalls durch ein oder zwei Halogenatome substituiert ist, steht.

2. Ester nach Anspruch 1 der Formel

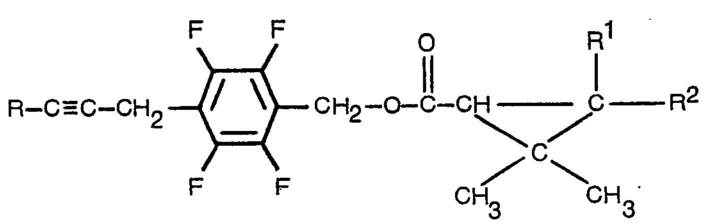

worin R für Wasserstoff oder Alkyl mit bis zu 4 Kohlenstoffatomen steht und $R^1$ und $R^2$ jeweils ausgewählt sind aus Halogen und Alkyl mit bis zu 4 Kohlenstoffatomen oder $R^1$ für Wasserstoff und $R^2$ für eine Gruppe der Formel

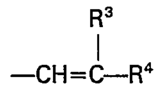

steht, worin $R^3$ und $R^4$ jeweils ausgewählt sind aus Methyl, Fluoro, Chloro, Bromo und Trifluoromethyl.

3. Ester nach Anspruch 2, bei welchem R für Wasserstoff, $R^1$ für Wasserstoff und $R^2$ für Dichlorovinyl oder 2-Chloro-3,3,3-trifluoroprop-1-en-1-yl steht.

4. Insektizide Zusammensetzung, welche eine insektizid wirksame Menge eines Esters nach Anspruch 1 gemeinsam mit einem insektizid inerten Verdünnungs- oder Trägermaterial enthält.

5. Verfahren zur Bekämpfung von Insektenschädlingen an einer bestimmten Stelle, bei welchem die Stelle mit einer insektizid wirksamen Menge der Zusammensetzung von Anspruch 4 behandelt wird.

6. Verfahren nach Anspruch 5, bei welchem die Insektenschädlinge fliegende Insektenschädlinge sind und die Zusammensetzung eine Menge der Verbindung von Anspruch 1 enthält, die ausreicht, die Schädlinge rasch zu betäuben.

7. Verfahren zur Herstellung des Esters von Anspruch 1, bei welchem eine Säure der Formel X—COOH oder ein veresterbares Derivat davon mit einem Alkohol der Formel

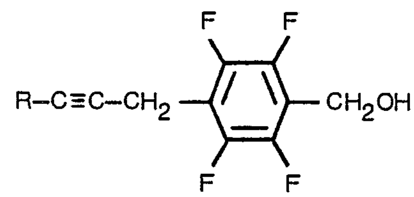

worin X und R die in Anspruch 1 angegebenen Bedeutungen besitzen, umgesetzt wird.

8. Alkohol der Formel

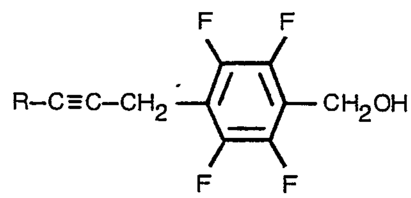

worin R für Wasserstoff oder Alkyl mit bis zu 4 Kohlenstoffatomen steht.

**Revendications**

1. Ester der formule

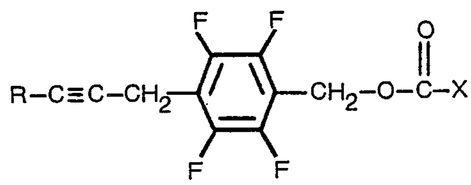

dans laquelle R est choisi entre l'hydrogène, un groupe alkyle ayant jusqu'à 4 atomes de carbone et un groupe trialkylsilyle dont chaque radical alkyle contient jusqu'à 4 atomes de carbone, et X représente

(a) un groupe de formule

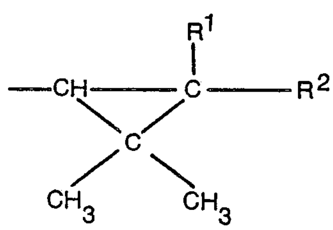

dans laquelle (i) $R^1$ et $R^2$ sont tous deux choisis entre l'hydrogène, un radical halogéno et un radical alkyle ayant jusqu'à 4 atomes de carbone; ou bien (ii) $R^1$ est l'hydrogène et $R^2$ représente un groupe de formule:

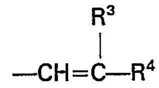

ou un groupe de formule:

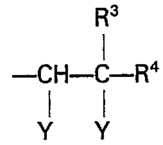

où $R^3$ et $R^4$ sont tous deux choisis entre les radicaux méthyle, halogéno et halogénalkyle ayant un ou deux atomes de carbone et contenant au moins deux atomes de fluor, et Y est le chlore ou le brome; ou bien

(b) X représente un groupe de formule:

$$Ar—CHR^5—$$

dans laquelle $R^5$ est un groupe alkyle ayant jusqu'à quatre atomes de carbone et Ar représente un groupe phényle facultativement substitué avec un ou deux atomes d'halogènes.

2. Ester suivant la revendication 1, de formule

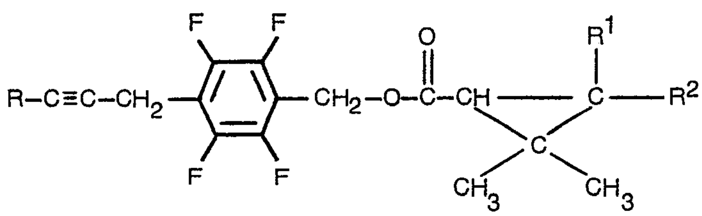

dans laquelle R est l'hydrogène ou un groupe alkyle ayant jusqu'à 4 atomes de carbone et $R^1$ et $R^2$ sont tous deux choisis entre un halogène et un groupe alkyle ayant jusqu'à 4 atomes de carbone, ou bien $R^1$ est l'hydrogène et $R^2$ est un groupe de formule:

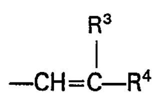

dans laquelle $R^3$ et $R^4$ sont tous deux choisis entre les radicaux méthyle, fluoro, chloro, bromo et trifluorométhyle.

3. Ester suivant la revendication 2, dans lequel R est l'hydrogène, $R^1$ est l'hydrogène et $R^2$ est un groupe dichlorovinyle ou 2-chloro-3,3,3-trifluoroprop-1-ène-1-yle.

4. Composition insecticide, comprenant une quantité efficace du point de vue insecticide de l'ester suivant la revendication 1 en association avec un diluant ou support inerte du point de vue insecticide.

5. Procédé pour combattre des insectes parasites en un lieu, qui consiste à traiter le lieu avec une quantité efficace du point de vue insecticide de la composition suivant la revendication 4.

6. Procédé suivant la revendication 5, dans lequel les insectes parasites sont des insectes parasites volants et la composition comprend une quantité du composé suivant la revendication 1 suffisante pour produire un effet de choc rapide sur les parasites.

7. Procédé de préparation de l'ester suivant la revendication 1, qui consiste à faire réagir un acide de formule X—COOH ou un dérivé estérifiable de cet acide, avec un alcool de formule:

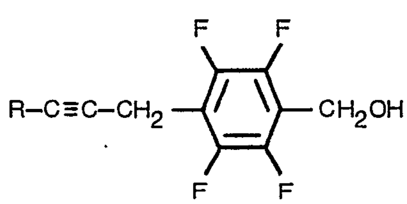

dans laquelle X et R sont tels que définis dans la revendication 1.

8. Alcool de formule:

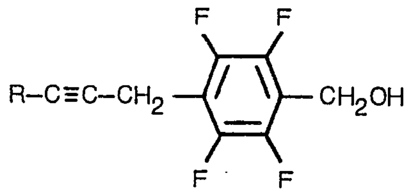

dans laquelle R est l'hydrogène ou un groupe alkyle ayant jusqu'à 4 atomes de carbone.